# EUROPEAN PATENT APPLICATION

(11) **EP 3 219 267 A1**
(43) Date of publication of application: **20.09.2017**
(21) Application number: 17157863.6
(22) Date of filing: 24.02.2017
(51) Int. Cl.: A61B 17/32, A61B 17/3203, A61M 1/00

(54) **SURGICAL HANDPIECE, LIQUID EJECTING APPARATUS, AND ASPIRATION APPARATUS**

(30) Priority: 29.02.2016 JP 2016036798
(71) Applicant: Seiko Epson Corporation, Tokyo 160-8801 (JP)
(72) Inventor: Yamasaki, Sosuke, Nagano 392-8502 (JP); Kurihara, Toru, Nagano 392-8502 (JP)
(74) Representative: Hoffmann Eitle

(57) **Abstract**

A surgical handpiece includes a housing gripped by a user, and an aspiration pipe that is attachably and detachably held by the housing and aspirates a liquid.

## Description

### BACKGROUND

### 1. Technical Field

The present invention relates to surgery using a liquid.

### 2. Related Art

A liquid ejecting apparatus is known as a surgical apparatus for resecting biological tissues via a liquid. A liquid ejecting apparatus disclosed in JP-A-2015-58169 includes an aspirating flow path member for aspirating a liquid or resected tissues in addition to a supply pipe for ejecting the liquid. The aspirating flow path member disclosed in JP-A-2015-58169 is a pipe that protrudes from a housing, and is formed of one tube.

A liquid ejecting apparatus does not necessarily require an aspirating flow path member to resect biological tissues. Accordingly, it is considered that it may be better for the aspirating flow path member not to include a portion protruding from a surgical handpiece depending on the state of a surgical site or the usage state of the apparatus. In contrast, in the liquid ejecting apparatus disclosed in JP-A-2015-58169, the aspirating flow path member is formed of one pipe, and the liquid ejecting apparatus does not take into full consideration such a demand.

The aforementioned description is common to apparatuses for surgery using a liquid. Examples of the apparatuses include a surgical apparatus that uses ultrasonic waves for breaking up, and an apparatus that aspirates a liquid in surgery using a liquid.

### SUMMARY

An advantage of some aspects of the invention is to improve the ease of use of an aspiration member of a surgical handpiece.

The advantage can be attained by the following configurations.

An aspect of the invention is directed to a surgical handpiece including a housing gripped by a user; and an aspiration pipe that is attachably and detachably held by the housing, and aspirates a liquid. According to this aspect, the aspiration pipe which is an aspiration member is attachable and detachable, and thus the ease of use is improved.

In the aspect, the housing may include a ring member in which a through hole is formed, and an inserted portion which is a portion of the aspiration pipe may be inserted into the through hole such that the aspiration pipe is mounted to the housing. According to this aspect, it is possible to mount the aspiration pipe only by inserting the inserted portion into the ring member, and it is possible to detach the aspiration pipe only by pulling the inserted portion out of the ring member.

In the aspect, the diameter of an inscribed circle, which is virtually in contact with the through hole which is projected to a plane perpendicular to an inserting direction of the inserted portion in a state where the aspiration pipe is detached, may be smaller than that of the inserted portion. According to this aspect, screws, an adhesive, or the like are not required, and thus it is possible to realize the aforementioned aspect with a simple configuration.

In the aspect, the through hole on the projected plane may include an arc and two straight lines which are respectively connected to both ends of the arc, and the inscribed circle may be in contact with the arc and the two straight lines. According to this aspect, contact between the inserted portion and the ring member is stabilized. The reason for this is that if three points are determined, the shape of a circle is determined. If contact between the inserted portion and the ring member is stable, the mounted aspiration pipe is stably held.

In the aspect, the through hole on the projected plane may include an indented surface that is connected to ends of the two straight lines and is indented toward an outer peripheral surface of the ring member. According to this aspect, when the inserted portion is inserted, the through hole is easily widened. For this reason, occurrence of an excessive force during insertion is prevented.

In the aspect, an end surface of the inserted portion in the inserting direction may be chamfered. According to this aspect, the inserted portion is easily inserted into the ring member.

In the aspect, the aspiration pipe may include a flange which has an outer diameter larger than that of the inserted portion, and which is in contact with the housing in a state where the aspiration pipe is mounted to the housing. According to this aspect, it is possible to determine whether the aspiration pipe is correctly mounted, based on the exterior.

In the aspect, the surgical handpiece may include a supply pipe configured to supply the liquid to a target resection site. According to this aspect, it is possible to supply a liquid for surgery using a liquid.

In the aspect, the supply pipe may intermittently eject the liquid. According to this aspect, it is possible to perform surgery via intermittent ejection of the liquid.

The invention can be realized in various forms other than the aforementioned aspects. For example, it is possible to realize a liquid ejecting apparatus or an aspiration apparatus which includes the aforementioned handpiece.

### BRIEF DESCRIPTION OF THE DRAWINGS

The invention will be described with reference to the accompanying drawings, wherein like numbers reference like elements.
Fig. 1 is a view illustrating a schematic configuration of a liquid ejecting apparatus.
Fig. 2 is a perspective view of a handpiece.
Fig. 3 is a side view of the handpiece.
Fig. 4 is a sectional view of the handpiece.
Fig. 5 is an enlarged sectional view illustrating the vicinity of a drive unit.
Fig. 6 is an enlarged sectional view of the vicinity of a diaphragm.
Fig. 7 is an enlarged sectional view of a region in which a supply pipe and an aspirating flow path member branch from each other.
Fig. 8 is a perspective view illustrating a state in which an aspiration pipe is detached from a housing in the handpiece.
Fig. 9 is a sectional view of the aspiration pipe.
Fig. 10 is an enlarged view of a connection portion illustrated in Fig. 8.
Fig. 11 is a view of the connection portion viewed from a positive side toward a negative side in an X2 direction.
Fig. 12 is a view illustrating fitting between an inner peripheral surface and an inserted portion of the aspiration pipe.
Fig. 13 is an enlarged view of the vicinity of a contact portion illustrated in Fig. 12.
Fig. 14 is a view illustrating the exterior of the handpiece in a disassembled state.
Fig. 15 is a view illustrating a state in which a second housing is detached from a first housing.
Fig. 16 is a perspective view illustrating the vicinity of a protruding portion of the second housing.
Fig. 17 is a perspective view illustrating the vicinity of a protruding portion of the second housing.
Fig. 18 is a perspective view illustrating the vicinity of a ring member of the first housing.
Fig. 19 is a view illustrating a state in which the aspirating flow path member is detached from the first housing.
Fig. 20 is a sectional view of the aspiration pipe and the handpiece which are disassembled from each other.
Fig. 21 is a sectional view of the handpiece in a state where the aspiration pipe is mounted to the handpiece.
Fig. 22 is a sectional view of a aspiration pipe and the handpiece which are disassembled from each other (Modification Example 1).
Fig. 23 is a sectional view of the aspiration pipe and the handpiece which are mounted to each other (Modification Example 1).
Fig. 24 is a view of the connection portion viewed from the positive side toward the negative side in the X2 direction (Modification Example 2).
Fig. 25 is a sectional view of the vicinity of the connection portion of a handpiece (Modification Example 2).

### DESCRIPTION OF EXEMPLARY EMBODIMENTS

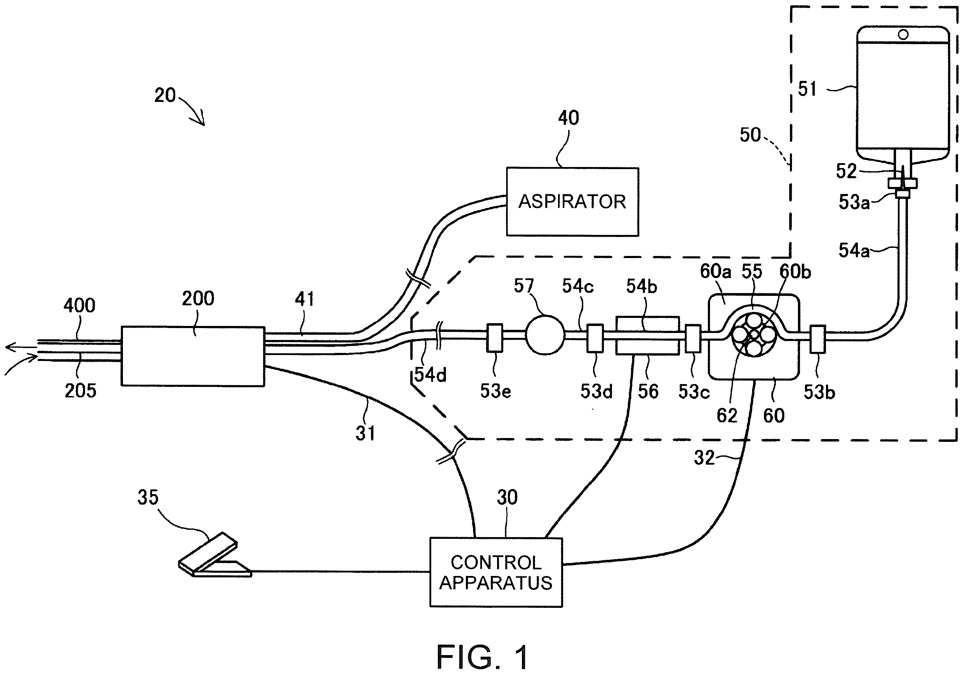

Fig. 1 illustrates a schematic configuration of a surgical apparatus 20. The surgical apparatus 20 is a medical device for realizing surgery using a liquid. The surgical apparatus 20 has the function of resecting a target lesion (biological tissues) by ejecting a liquid to the target lesion, and the function of aspirating the ejected liquid or the resected biological tissues. For this reason, the surgical apparatus 20 is a liquid ejecting apparatus and an aspiration apparatus.

The surgical apparatus 20 includes a control apparatus 30; an actuator cable 31; a pump cable 32; a foot switch 35; an aspirator 40; an aspiration tube 41; a liquid supply apparatus 50; and a surgical handpiece 200 (hereinafter, referred to as a handpiece 200).

The liquid supply apparatus 50 includes a water supply bag 51; a spike needle 52; first to fifth connectors 53a to 53e; first to fourth water supply tubes 54a to 54d; a pump tube 55; a blockage detection mechanism 56; a filter 57; and a tube pump 60. The handpiece 200 includes a supply pipe 205 and an aspiration pipe 400.

The water supply bag 51 is made of transparent synthetic resin, and the inside of the water supply bag 51 is filled with a liquid (specifically, a physiological salt solution). In this application, even if a liquid supply bag is filled with liquids other than water, the liquid supply bag is referred to as the water supply bag 51. The spike needle 52 is connected to the first water supply tube 54a via the first connector 53a. If the spike needle 52 punctures the water supply bag 51, the liquid with which the water supply bag 51 is filled can be supplied to the first water supply tube 54a.

The first water supply tube 54a is connected to the pump tube 55 via the second connector 53b. The pump tube 55 is connected to the second water supply tube 54b via the third connector 53c. The pump tube 55 is interposed between a stator 60a and a rotor 60b of the tube pump 60. The tube pump 60 squeezes the pump tube 55 by rotating multiple rollers on the rotor via rotating of a built-in motor (supply motor 62). Liquid inside of the pump tube 55 is fed from a first water supply tube 54a side to a second water supply tube 54b side by squeezing. The rotating of the supply motor 62 implies that "the tube pump 60 is driven".

The blockage detection mechanism 56 detects blockage of a flow path inside of the second to fourth water supply tubes 54b to 54d and the handpiece 200 by measuring pressure inside of the second water supply tube 54b. If the blockage detection mechanism 56 detects blockage, the blockage detection mechanism 56 inputs a detection result into the control apparatus 30.

The second water supply tube 54b is connected to the third water supply tube 54c via the fourth connector 53d. The filter 57 is connected to the third water supply tube 54c. The filter 57 collects foreign matters contained in the liquid.

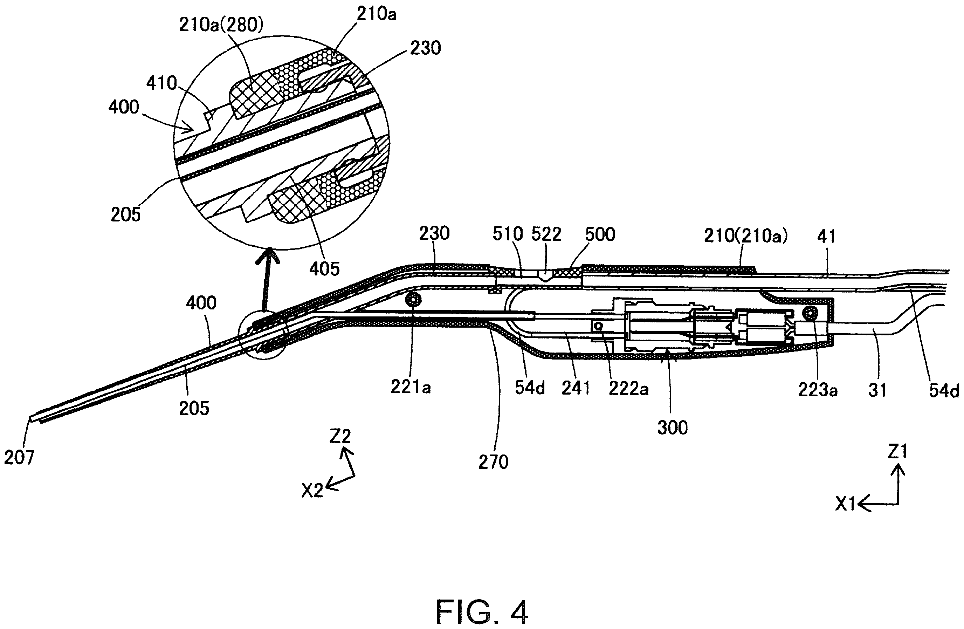

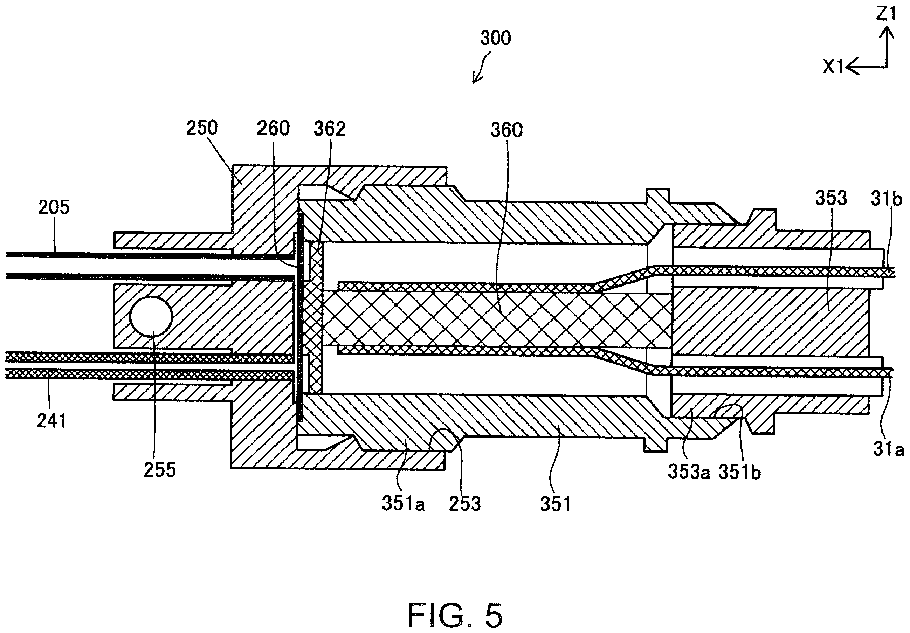

The third water supply tube 54c is connected to the fourth water supply tube 54d via the fifth connector 53e. The fourth water supply tube 54d is connected to the handpiece 200. The liquid supplied to the handpiece 200 via the fourth water supply tube 54d is intermittently ejected from a nozzle 207 provided at a distal end of the supply pipe 205 by the driving of a drive unit 300 (refer to Figs. 4 and 5). It is possible to ensure resecting capability with a small amount of flow rate by intermittently ejecting the liquid as such. The supply pipe 205 is a pipe for supplying the liquid for resecting biological tissues to a target resection site. Since the supply pipe 205 is a pipe for ejecting the liquid, the supply pipe 205 is also referred as an ejection pipe. The supply pipe 205 is also referred to as a pipe through which a liquid used in surgery flows.

The aspiration tube 41 is connected to the handpiece 200. The aspirator 40 aspirates substances inside of the aspiration pipe 400 via the aspiration tube 41. The liquid, resected pieces, and the like in the vicinity of a distal end of the aspiration pipe 400 are aspirated by the aspiration. Similar to the supply pipe 205, the aspiration pipe 400 is a pipe through which the liquid used in surgery flows.

The control apparatus 30 includes a central processing unit (CPU) and a storage medium. The storage medium stores a program for controlling the tube pump 60 and a piezoelectric element 360 (refer to Fig. 5). While a user depressed the foot switch 35, the control apparatus 30 transmits a first drive signal via the actuator cable 31 and a second drive signal via the pump cable 32 by executing the program via the CPU. In the embodiment, the first drive signal has a frequency of 400 Hz, and drives the drive unit 300. The second drive signal drives the tube pump 60. Accordingly, while a user depresses the foot switch 35, the liquid is intermittently ejected, and while the user does not depress the foot switch 35, ejection of the liquid stops.

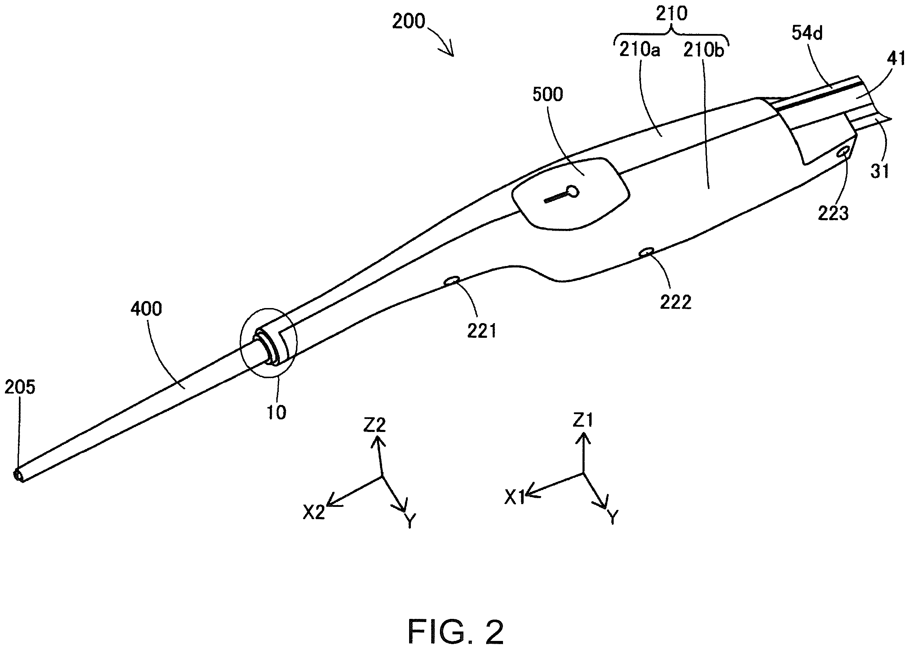

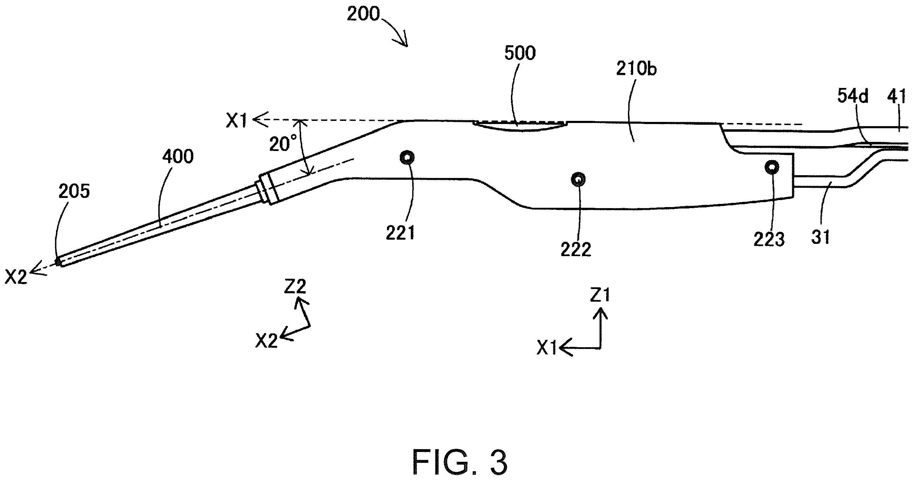

Fig. 2 is a perspective view of the handpiece 200. Fig. 3 is a side view of the handpiece 200. The handpiece 200 includes the supply pipe 205; a housing 210; screws 221, 222, and 223; the aspiration pipe 400; and an aspirating force adjustment mechanism 500.

The housing 210 is a member gripped by a user. The housing 210 is formed by joining together a first housing 210a and a second housing 210b. The supply pipe 205 is made of metal, and has conductivity. The housing 210 and the aspiration pipe 400 are made of hard resin.

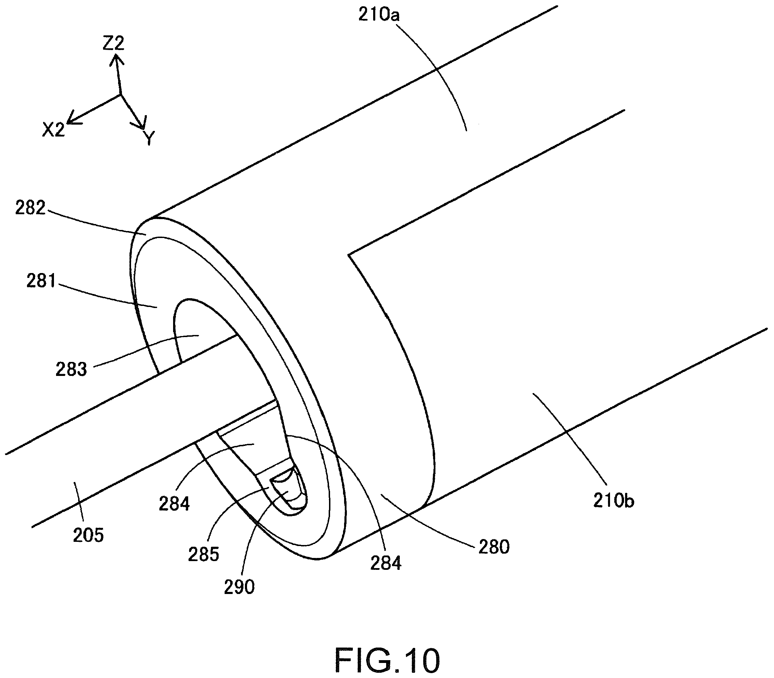

As illustrated in Figs. 2 and 3, two rectangular coordinate systems are defined with respect to the handpiece 200. A first one is an X1-Y-Z1 coordinate system. A second one is an X2-Y-Z2 coordinate system. A Y direction common to the two coordinate systems is a direction perpendicular to a boundary line between the first housing 210a and the second housing 210b, and is a positive direction oriented from the first housing 210a toward the second housing 210b. As illustrated in Fig. 2, the boundary line is a line illustrated on a surface of the housing 210. In the definition of the Y direction, the vicinity of a connection portion 10 is apart from the boundary line. The connection portion 10 will be described later with reference to Fig. 10.

An X1 direction is a direction parallel to a predetermined straight line included in the boundary line. A positive side in the X1 direction is oriented toward an opening end of the aspiration pipe 400. The predetermined straight line is a straight line illustrated on both sides of the aspirating force adjustment mechanism 500. A Z1 direction is defined from the X1 direction and the Y direction by a right-handed system.

An X2 direction is a longitudinal direction of the aspiration pipe 400. A positive side in the X2 direction is oriented toward to the opening end of the aspiration pipe 400. A Z2 direction is defined from the X2 direction and the Y direction by a right-handed system. In the embodiment, the angle between the X1 direction and the X2 direction is 20 degrees.

The second housing 210b is fixed to the first housing 210a by tightening the screws 221, 222, and 223. Among the screws 221, 222, and 223, the screw 223 is positioned farthest to the negative side in the X1 direction. The position of the screw 223 is disposed in the vicinity of a negative side end portion of the housing 210 in the X1 direction. For this reason, the second housing 210b is firmly fixed to the first housing 210a in the vicinity of the negative side end portion of the housing 210 in the X1 direction.

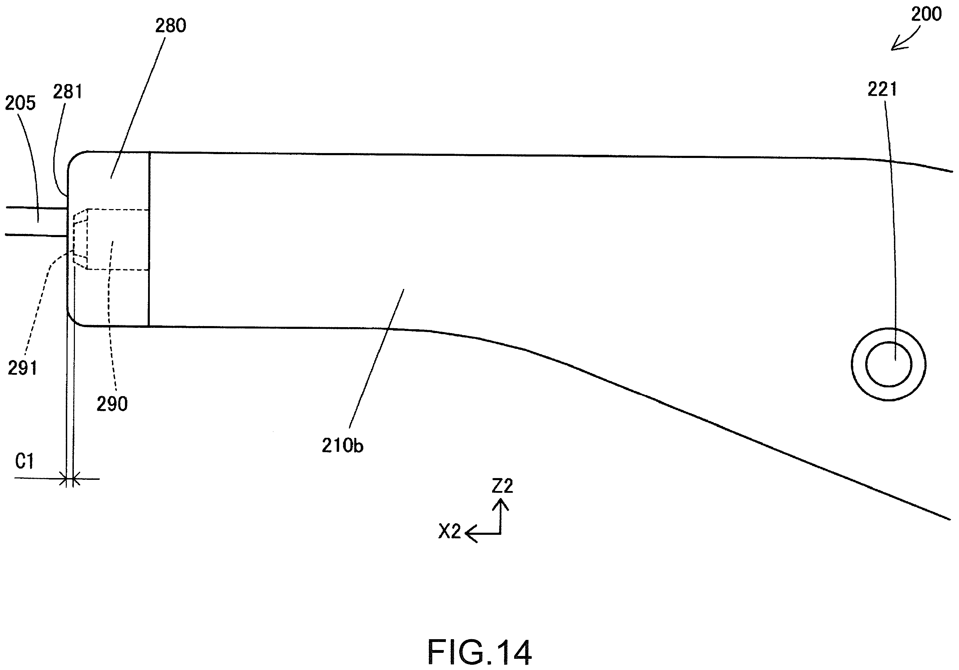

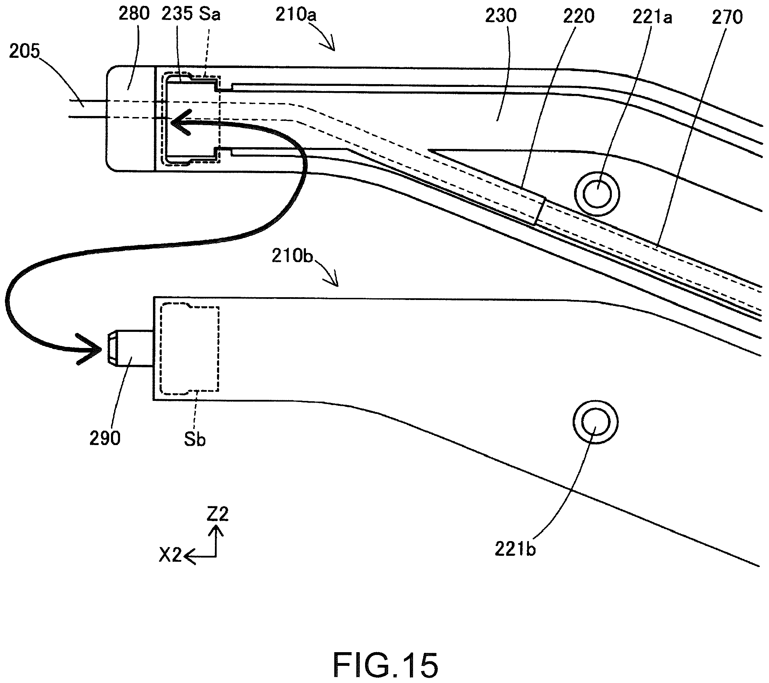

Among the screws 221, 222, and 223, the screw 221 is positioned farthest to the positive side in the X1 direction. The position of the screw 221 is close to a positive side end portion of the housing 210 in the X1 direction, that is, is closer to the negative side in the X1 direction than the vicinity of the connection portion 10. The reason for this is to design a portion of the housing 210, which is closer to the positive side in the X1 direction than the screw 221, to be thin. The second housing 210b is also firmly fixed to the first housing 210a in a region which is closer to the positive side in the X1 direction than the screw 221 (to be described later with reference to Figs. 14 and 15).

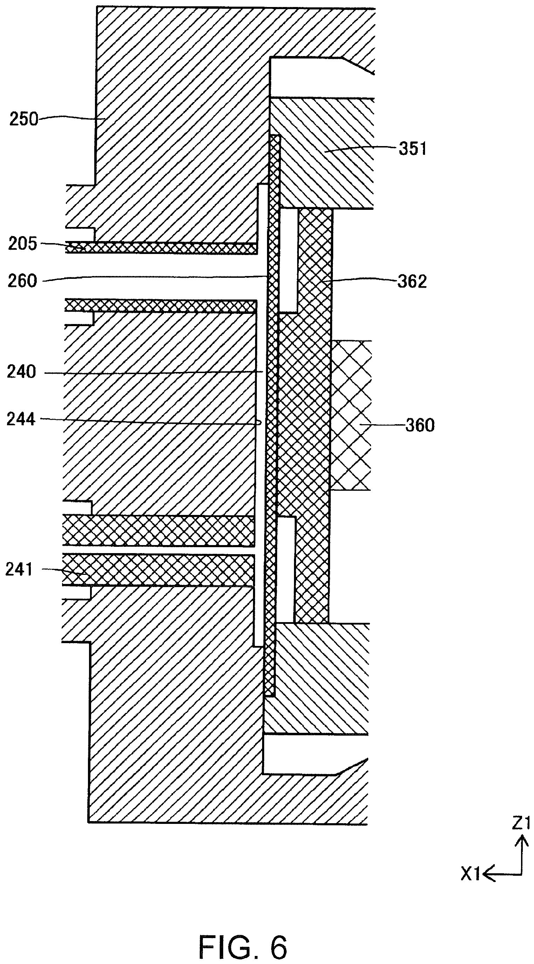

Fig. 4 is a sectional view of the handpiece 200. Screw holes 221a, 222a, and 223a, an inlet flow path 241, an insulative member 270 (to be described later with reference to Fig. 6), and the drive unit 300 are provided inside of the housing 210.

A surface illustrated as a sectional surface of the housing 210 in Fig. 4 is a mating surface of the first housing 210a. The mating surface of the first housing 210a is a surface perpendicular to the Y direction, and is a surface that is in contact with the second housing 210b when the first housing 210a is assembled into the housing 210. A region, which is illustrated as a ring member 280 (to be described later) in a circled enlarged view, is not a mating surface, and is a cut surface of the ring member 280. The supply pipe 205 and the aspiration pipe 400 pass through the ring member 280.

The fourth water supply tube 54d is bent into a U shape inside of the housing 210, and is connected the inlet flow path 241. The inlet flow path 241 communicates with the supply pipe 205 via a liquid chamber 240 (refer to Fig. 6).

The flow path diameter of the inlet flow path 241 is smaller than that of the supply pipe 205. For this reason, even pressure inside of the liquid chamber 240 changes (to be described later), the liquid inside of the liquid chamber 240 is prevented from flow back to the inlet flow path 241.

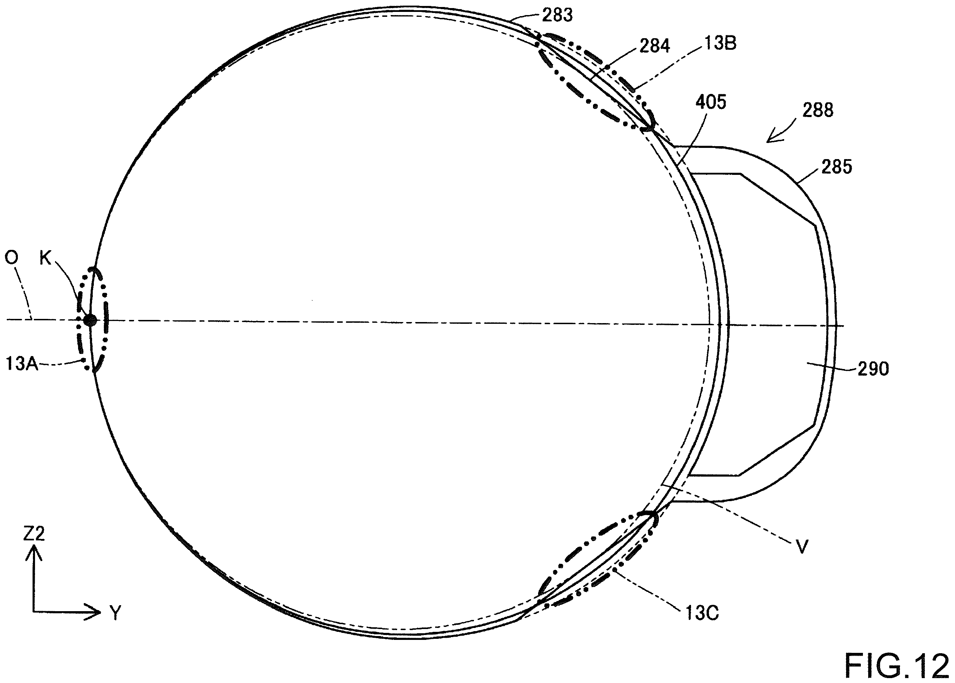

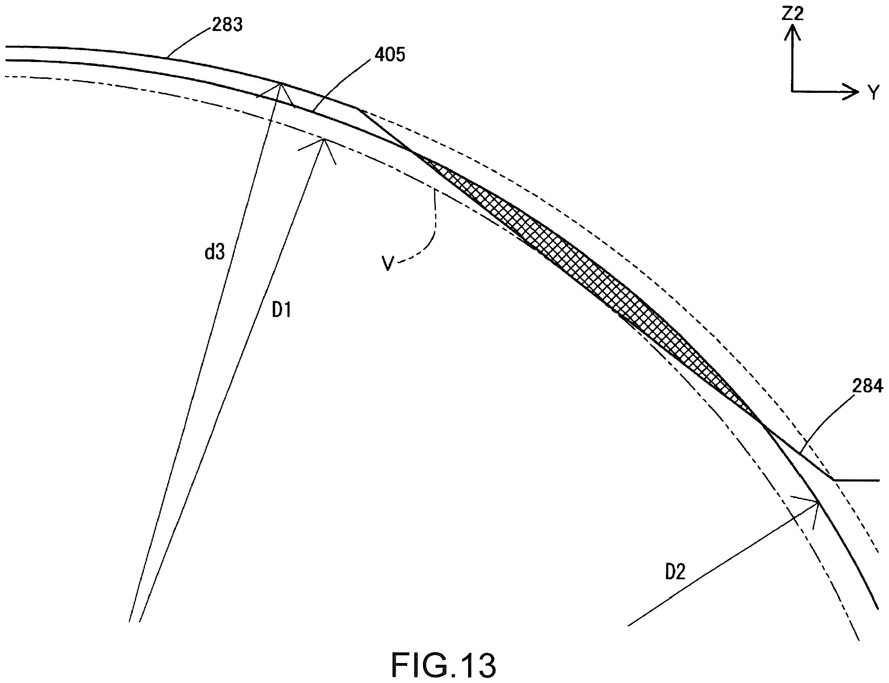

The housing 210 includes the ring member 280. The mounting of the aspiration pipe 400 is realized by fitting an inserted portion 405, which is a portion of the aspiration pipe 400, to the ring member 280 (to be described later with reference to Figs. 12 and 13), and bringing a flange 410, which is a portion of the aspiration pipe 400, into contact with the ring member 280 of the first housing 210a. A flow path inside of the mounted aspiration pipe 400 communicates with an aspirating flow path member 230. The aspirating flow path member 230 is more flexible than the supply pipe 205, and is made of soft resin. The aspirating flow path member 230 is connected to the aspiration tube 41 via the aspirating force adjustment mechanism 500.

The aspirating force adjustment mechanism 500 is provided with an aspirating flow path member 510 through which the aspirating flow path member 230 is connected to the aspiration tube 41, and a hole 522 that communicates with a flow path inside of the aspirating flow path member 510. A user can adjust aspirating force through the aspiration pipe 400 using the hole 522. Specifically, if the opening area of the hole 522 is decreased, the flow rate of air flowing through the hole 522 decreases, and thus, the flow rate of a fluid (air, liquid, and the like) aspirated via the aspiration pipe 400 increases. That is, aspirating force through the aspiration pipe 400 increases. In contrast, if the opening area of the hole 522 is increased, the flow rate of air flowing through the hole 522 increases, and thus, aspirating force through the aspiration pipe 400 decreases. Typically, a user realizes adjustment of the opening area of the hole 522 by adjusting the area of the hole 522 blocked by the thumb. The shape of the hole 522 is designed such that aspirating force through the aspiration pipe 400 is very small, or aspirating force is not applied at all in a state where the hole 522 is not covered at all. In the embodiment, the flow path area of the aspiration pipe 400 is larger than the opening area of the hole 522, but the length of the aspiration pipe 400 is sufficiently long. Therefore, flow path resistance of the aspiration pipe 400 is higher than that of the hole 522. It is possible to decrease aspirating force through the aspiration pipe 400 to a very small level if the hole 522 is not covered at all in this manner.

The screw holes 221a, 222a, and 223a are provided in the first housing 210a. The screws 221, 222, and 223 are respectively screwed into the screw holes 221a, 222a and 223a. The screw 222 passes through a hole 255 (refer to Fig. 5) provided in a flow path connection member 250 of the drive unit 300, and is inserted into the screw hole 222a.

Fig. 5 is an enlarged sectional view illustrating the vicinity of the drive unit 300. The drive unit 300 includes the flow path connection member 250; a diaphragm 260; a circular cylindrical member 351; a fixing member 353; the piezoelectric element 360; and a piston 362.

The piezoelectric element 360 is a laminated piezoelectric element. The piezoelectric element 360 is disposed inside of the circular cylindrical member 351 such that an expanding and contracting direction of the piezoelectric element is positioned along the X1 direction.

The fixing member 353 is fixed to one end of the circular cylindrical member 351. Specifically, a male screw 353a provided on an outer periphery of the fixing member 353 is tightened to a female screw 351b provided on an inner periphery of the circular cylindrical member 351, and thus the fixing member 353 is fixed to the one end of the circular cylindrical member 351.

The piezoelectric element 360 is fixed to the fixing member 353 via an adhesive. A first cable 31a and a second cable 31b of the actuator cable 31 pass through a through hole provided in the fixing member 353, and are electrically connected to the piezoelectric element 360.

The material of the diaphragm 260 is metal, and specifically, is stainless steel, and more specifically, SUS304 or SUS316L. The diaphragm 260 is formed into a thickness (for example, 300 µm) such that a preload is applied to the piezoelectric element 360. The diaphragm 260 is disposed to cover the other end of the circular cylindrical member 351, and is fixed to the circular cylindrical member 351 via welding.

The piston 362 is fixed to one end of the piezoelectric element 360 via an adhesive, and is disposed in such a way as to be in contact with the diaphragm 260. The piston 362 has a shape in which circular columns having different diameters are concentrically stacked on each other. The circular column having a smaller diameter is in contact with the diaphragm 260. For this reason, ends of the diaphragm 260 are not pressed, and large force is not applied to welded locations. There is no fixation between the piston 362 and the diaphragm 260 via an adhesive or the like, and there is only contact therebetween.

A male screw 351a is provided on an outer periphery of the circular cylindrical member 351. The male screw 351a is tightened to a female screw 253 provided in the flow path connection member 250, and thus the circular cylindrical member 351 is fixed to the flow path connection member 250.

The piezoelectric element 360 expands and contracts in response to a drive signal input via the actuator cable 31. If the piezoelectric element 360 expands and contracts, the piston 362 vibrates in a longitudinal direction (the X1 direction) of the piezoelectric element 360. If the piston 362 vibrates, the diaphragm 260 is deformed in compliance with the vibration. The expanding and contracting of the piezoelectric element 360 in correspondence with a drive signal implies that the drive unit 300 is driven.

Fig. 6 is an enlarged section illustrating the vicinity of the diaphragm 260. A recess 244 is provided in the flow path connection member 250. The recess 244 is a portion of the flow path connection member 250, which is shallowly recessed into a circular shape. The recess 244 is covered with the diaphragm 260, and thus the liquid chamber 240 is formed in the recess 244. The liquid chamber 240 formed in this manner is capable of communicating the supply pipe 205 for ejecting the liquid.

If the diaphragm 260 is deformed, the volume of the liquid chamber 240 is changed. The pressure of the liquid with which the liquid chamber 240 is filled changes due to this change. When pressure inside of the liquid chamber 240 decreases, the liquid flows into the liquid chamber 240 from the inlet flow path 241. When pressure inside of the liquid chamber 240 increases, the liquid flows out of the liquid chamber 240 to the supply pipe 205. As described above, the reason for the liquid flowing in such directions is that the flow path diameter of the inlet flow path 241 is narrower than that of the supply pipe 205.

The liquid which has flowed to the supply pipe 205 is ejected from the distal end of the supply pipe 205. Since pressure inside of the liquid chamber 240 intermittently increases, the liquid is intermittently ejected from the supply pipe 205.

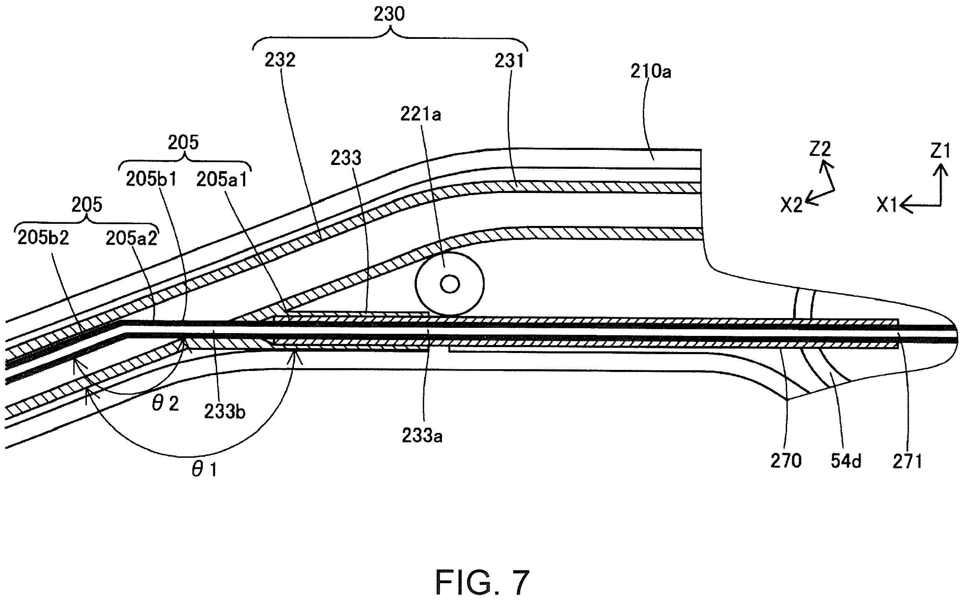

Fig. 7 is an enlarged sectional view of a region in which the supply pipe 205 and the aspirating flow path member 230 branch from each other. A hole 233b opens in a wall of the aspirating flow path member 230. The supply pipe 205 is disposed to pass through the hole 233b. Due to this disposition, a portion of the supply pipe 205 and a portion of the aspirating flow path member 230 form a two-layer pipe. An inner pipe portion 205b1 refers to a portion of the supply pipe 205 which is an inner pipe of the two-layer pipe formed of the supply pipe 205 and the aspirating flow path member 230, that is, a range from a portion of the supply pipe 205, which is closer to the positive side in the X1 direction than the hole 233b, to a positive side distal end of the aspirating flow path member in the X2 direction. A branched portion 205a1 refers to a portion of the supply pipe 205 which is closer to the negative side in the X1 direction than the hole 233b, that is, a portion of the supply pipe 205 which branches from the aspirating flow path member 230. Since the supply pipe 205 includes the inner pipe portion 205b1, the supply pipe 205 is also referred to as an inner pipe member.

The supply pipe 205 can be also divided into a rear end side 205a2 and a distal end side 205b2. The rear end side 205a2 is a portion of the supply pipe 205 which extends in the X1 direction. The distal end side 205b2 is a portion of the supply pipe 205 which extends in the X2 direction. The distal end side 205b2 is positioned on the positive side in the X1 direction and on a positive side in the X2 direction relative to the rear end side 205a2.

Since the rear end side 205a2 passes through the hole 233b, the boundary between the rear end side 205a2 and the distal end side 205b2 is disposed inside of a distal end side 232. For this reason, the supply pipe 205 includes a portion that is the inner pipe portion 205b1 and the rear end side 205a2.

The aspirating flow path member 230 can be also divided into a rear end side 231 and the distal end side 232. The distal end side 232 is a portion that extends in the X2 direction. The rear end side 231 is a portion that extends in the X1 direction. The distal end side 232 is positioned on the positive side in the X1 direction and on the positive side in the X2 direction relative to the rear end side 231. Since a portion of the aspirating flow path member 230 forms a two-layer pipe together with the inner pipe portion 205b1, the aspirating flow path member 230 is also referred to as an outer pipe member.

The aspirating flow path member 230 includes a holding portion 233. The holding portion 233 is a pipe-shaped member branching from a main portion of the aspirating flow path member 230 which serves as a flow path. A wall of the holding portion 233 is connected to the distal end side 232 in such a way as to surround the hole 233b. Specifically, the holding portion 233 is molded integrally with the aspirating flow path member 230 via resin molding. Since the aspirating flow path member 230 and the holding portion 233 are made of soft resin, the holding portion 233 is an elastic member. The holding portion 233 extends perfectly straight from the periphery of the hole 233b to the negative side in the X1 direction. An open end 233a as a negative side end portion in the X1 direction is provided in the holding portion 233.

The insulative member 270 is disposed between the branched portion 205a1 and the holding portion 233. Since the insulative member 270 is made of high-density polyethylene, the insulative member 270 has insulating properties. The insulative member 270 is a pipe-shaped member that forms a three-layer pipe structure together with the holding portion 233 and the branched portion 205a1. The insulative member 270 is a second pipe (that is, a second pipe from the inside) from the outside of the three-layer pipe.

The insulative member 270 is disposed inside of the holding portion 233 by being inserted from the open end 233a of the holding portion 233. The insulative member 270 is longer than the holding portion 233. Accordingly, a portion of the insulative member 270 protrudes to the negative side in the X1 direction further from the holding portion 233. A negative side end portion 271 of the insulative member 270 in the X1 direction is positioned closer to the negative side in the X1 direction than a portion of the insulative portion which intersects the fourth water supply tube 54d. The distance between the end portion 271 and the housing 210 is longer than that between a portion such as the open end 233a and the housing 210. For this reason, electricity as noise is prevented from affecting the piezoelectric element 360 via the housing 210 and the supply pipe 205.

The branched portion 205a1 and the insulative member 270 have a dimensional relationship which allows interference fitting therebetween. Similarly, the insulative member 270 and the holding portion 233 have a dimensional relationship which allows interference fitting therebetween. The branched portion 205a1 is held by the holding portion 233 via the insulative member 270. That is, the holding portion 233 is a member for holding the supply pipe 205. The holding portion 233 has much lower rigidity than that of the insulative member 270. Accordingly, if the insulative member 270 is inserted into the holding portion 233, deformation of the holding portion 233 is greater than that of the insulative member 270. Radial deformation of the holding portion 233 is approximately 20% of a wall thickness of the holding portion 233.

As described above, the branched portion 205a1 and the insulative member 270 have a dimensional relationship which allows interference fitting therebetween, and thus a gap between the branched portion 205a1 and the insulative member 270 is sealed. Similarly, the insulative member 270 and the holding portion 233 have a dimensional relationship which allows interference fitting therebetween, and thus a gap between the insulative member 270 and the holding portion 233 is sealed. Accordingly, there is almost no leak of a fluid from a gap between the hole 233b and the rear end side 205a2.

As described above, the branched portion 205a1 is held by the holding portion 233, and thus substantially parallelism between the holding portion 233 and the branched portion 205a1 is maintained. In contrast, an angle θ1 formed by the distal end side 232 and the holding portion 233 is substantially the same as an angle θ2 formed by the rear end side 205a2 and the distal end side 205b2. For this reason, the distal end side 205b2 is held while being substantially parallel to the distal end side 232. In the embodiment, each of the angle θ1 and the angle θ2 is designed to be 160 degrees.

As described above, the branched portion 205a1 is held by the holding portion 233 which is an elastic member, and thus vibration of the supply pipe 205 is absorbed by the holding portion 233. The supply pipe 205 vibrates due to the driving of the piezoelectric element 360 or received force from the nozzle 207.

The screw hole 221a is disposed closer to the negative side in the X1 direction than the hole 233b, and is disposed between the branched portion 205a1 and the aspirating flow path member 230 in the Z1 direction. For this reason, in designing of the housing 210, a portion of the housing 210, which is closer to the positive side in the X2 direction than the inner pipe portion 205b1, can be thinned out to the extent that the portion of the housing 210 is capable of accommodating the distal end side 232.

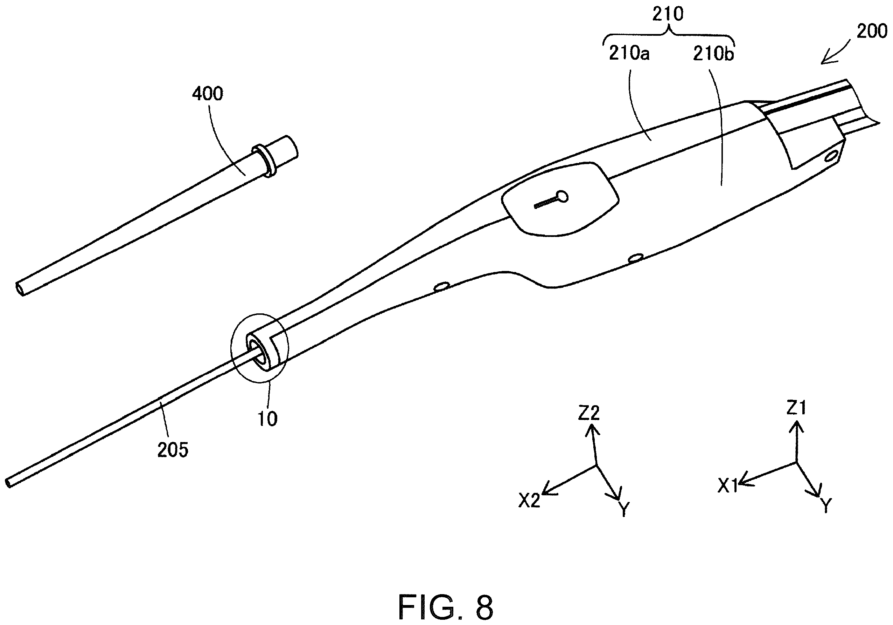

Fig. 8 is a perspective view illustrating a state in which the aspiration pipe 400 is detached from the housing 210 in the handpiece 200. Hereinafter, this state is referred to as a disassembled state. In contrast, as illustrated in Fig. 2 and the like, a state in which the aspiration pipe 400 is mounted in the housing 210 is referred to as a mounting state. The aspiration pipe 400 is formed such that a user can manually attach the aspiration pipe 400 to and detach the aspiration pipe 400 from the housing 210. A user may use the handpiece 200 in a disassembled state depending on the state of a surgical site or the usage state of the apparatus. If the handpiece 200 is in a disassembled state, the surgical apparatus 20 cannot be used as an aspiration apparatus. Hereinafter, a structure related to attachment and detachment of the aspiration pipe 400 will be described.

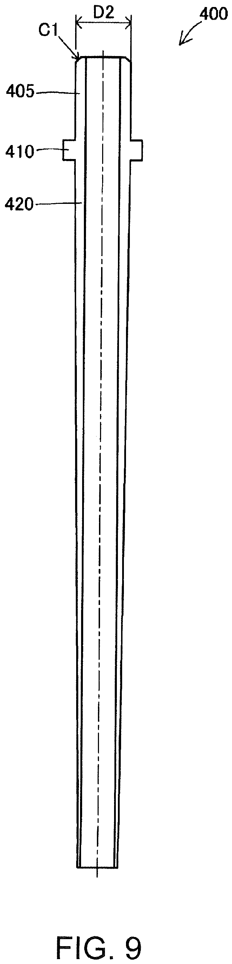

Fig. 9 is a sectional view of the aspiration pipe 400. The aspiration pipe 400 includes the inserted portion 405; the flange 410; and a main portion 420. As described with reference to Fig. 4, the inserted portion 405 is a portion of the aspiration pipe 400 which is inserted into the ring member 280. As illustrated in Fig. 9, a distal end surface of the inserted portion 405 is chamfered. The inserted portion 405 can be easily inserted into the ring member 280 due to the chamfer. The flange 410 is a portion of the aspiration pipe 400, the outer diameter of which is larger than that of the inserted portion 405.

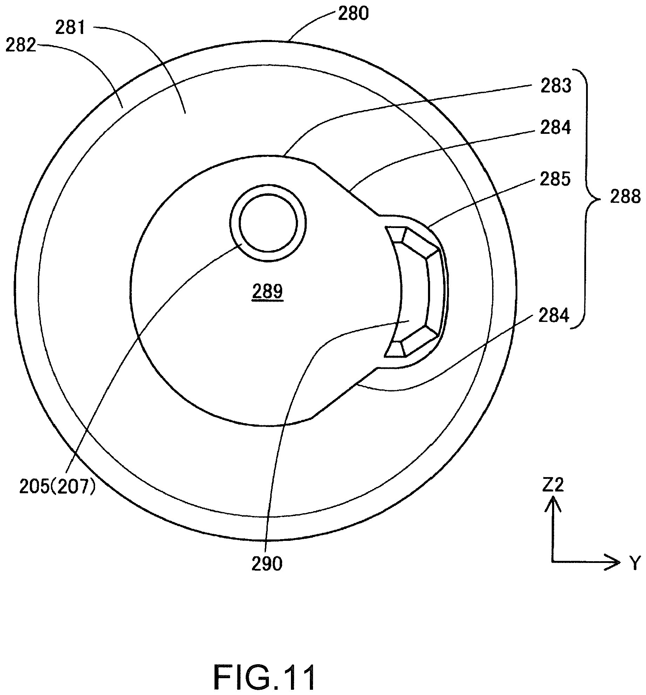

Fig. 10 is an enlarged view of the connection portion 10 in Fig. 8. Fig. 11 is a view of the connection portion 10 viewed from the positive side toward the negative side in the X2 direction. The first housing 210a and the second housing 210b have contour shapes which are substantially symmetrical to each other. The first housing 210a and the second housing 210b have contours which are asymmetrical to each other in the connection portion 10.

The first housing 210a includes the ring member 280 described with reference to Fig. 4. The ring member 280 includes a distal end surface 281; an R portion 282; an arc surface 283; two flat surfaces 284; and an indented surface 285.

The distal end surface 281 is a flat surface parallel to a Y-Z2 plane, and is a portion of the ring member 280 which is positioned farthest to the positive side in the X2 direction. The R portion 282 is an R portion through which the distal end surface 281 is connected to an outer peripheral surface of the ring member 280.

The arc surface 283, the two flat surfaces 284, and the indented surface 285 form an inner peripheral surface 288 of a through hole 289. The arc surface 283 is a portion having an arc shape when projected to the Y-Z2 plane. The indented surface 285 is a portion which is indented outward further from the arc surface 283 in a radial direction such that a wall thickness of the ring member 280 is thin. The two flat surfaces 284 are flat surfaces through which the arc surface 283 is connected to the indented surface 285.

The second housing 210b includes a protruding portion 290. The protruding portion 290 is disposed to fill up a portion that is indented to the indented surface 285. The protruding portion 290 will be described in detail with reference to Figs. 14 to 17.

Fig. 12 is a view illustrating fitting between the inner peripheral surface 288 and the inserted portion 405 of the aspiration pipe 400. Fig. 13 is an enlarged view of the vicinity of a contact portion 13B illustrated in Fig. 12. In description of Figs. 12 and 13, the inner peripheral surface 288, the inserted portion 405, and the protruding portion 290 are treated as two-dimensional lines which are projected to the Y-Z2 plane. Figs. 12 and 13 illustrate only outer peripheral surfaces of the inserted portion 405 and the protruding portion 290.

In Figs. 12 and 13, the exact shapes of the inserted portion 405 and the inner peripheral surface 288 in a mounting state are not illustrated, and the shapes in a disassembled state are illustrated. Since the inserted portion 405 and the inner peripheral surface 288 are elastically deformed in transition from a disassembled state to a mounting state, the shapes in the mounting state are different from those in the disassembled state, which will be described later.

In Figs. 12 and 13, the inserted portion 405 is disposed relative to the inner peripheral surface 288 such that points K overlap each other and axes O of symmetry overlap each other. The point K is a point on the Y-Z2 plane which is positioned farthest to a negative side in the Y direction. The point K is defined for each of the inserted portion 405 and the inner peripheral surface 288. The axis O of symmetry is a straight line parallel to the Y direction, and is a straight line that passes through the point K and the centers of the arc surface 283 and the inserted portion 405.

As illustrated in Fig. 13, the inner diameter of the arc surface 283 is d3. As illustrated in Figs. 9 and 13, the outer diameter of the inserted portion 405 is D2. The diameter of a virtual inscribed circle V is D1. The virtual inscribed circle V is a virtual circle that passes through the points K and is in contact with the two flat surfaces 284. Each of the flat surfaces 284 is equivalent to a chord of a circle including the arc surface 283. Accordingly, the inner diameter d3 is larger than the diameter D1. The outer diameter D2 is designed to be smaller than the inner diameter d3, and to be larger than the diameter D1. For this reason, as illustrated in Figs. 12 and 13, a portion of each of the flat surfaces 284 overlaps the inserted portion 405. The overlapped portion is illustrated by hatching lines in Fig. 13. Accordingly, when the inserted portion 405 is inserted into a space defined by the inner peripheral surface 288, at least portions of the inserted portion 405 and the inner peripheral surface 288 illustrated by hatching lines are elastically deformed. The inserted portion 405 receives a combined force which is applied to the negative side in the Y direction and which is a combination of forces from the two flat surfaces 284. The combined force is applied to the vicinity of a portion 13A.

If the inserted portion 405 is inserted into the through hole 289, surface pressures are applied to the vicinity of the portions 13A, 13B, and 13C from the inner peripheral surface 288. The inserted portion 405 is held by the inner peripheral surface 288 due to frictional forces associated with the surface pressures.

As described above, the virtual inscribed circle V is in contact with the inner peripheral surface 288 at three points. If three points are determined, the shape of a circle is determined. Therefore, even if dimensional tolerances and the like are taken into consideration, the position and the diameter D1 of the virtual inscribed circle V are stable. Unlike the embodiment, if the virtual inscribed circle V is designed to be in contact with the inner peripheral surface 288 at four or more points, when dimensional tolerances and the like are taken into consideration, the number of contacts in an actual product may be reduced. If the number of contacts between the virtual inscribed circle V and the inner peripheral surface 288 is changed, the state of contact between the inner peripheral surface 288 and the inserted portion 405 becomes unstable. In the embodiment, the virtual inscribed circle V is in contact with the inner peripheral surface 288 at three points, and thus such a phenomenon may be avoided. Accordingly, the aforementioned frictional forces are stable. As a result, it is possible to realize design in which the aspiration pipe 400 in a mounting state can be stably held and the aspiration pipe 400 can be easily attached and detached.

Since the indented surface 285 is provided, deformability (spring properties) of the inner peripheral surface is ensured. That is, since the indented surface 285 is provided, when the inserted portion 405 is inserted, the through hole 289 is easily widened due to forces applied to the flat surfaces 284. For this reason, an excessive force does not occur when the inserted portion 405 is inserted into the through hole 289. As a result, insertion of the inserted portion 405 is easy.

Since the protruding portion 290 is disposed in a space formed by the indented surface 285, it is possible to insert the inserted portion 405 into the ring member 280 without causing interference between the inserted portion 405 and the protruding portion 290.

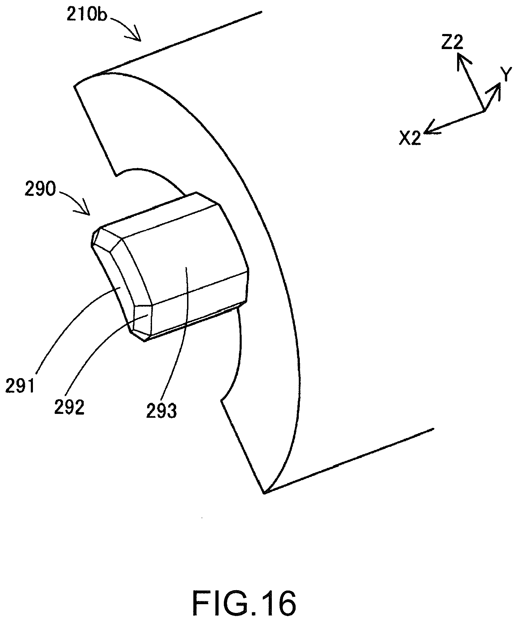

Fig. 14 illustrates the exterior of the handpiece 200 in a disassembled state. Since the protruding portion 290 cannot be seen in Fig. 14, the protruding portion 290 is illustrated by a dotted line. A distal end surface 291 (refer to Fig. 16) of the protruding portion 290 is positioned spaced by a clearance C1 from the distal end surface 281 of the ring member 280 to the negative side in the X2 direction. That is, the distal end surface 291 of the protruding portion 290 is recessed to the inside of the housing 210 further from the distal end surface 281 of the ring member 280 in the X2 direction. For this reason, there is no interference between the protruding portion 290 and the flange 410 in a mounting state.

Fig. 15 illustrates a state in which the second housing 210b is detached from the first housing 210a. If a user moves the screws 221, 222, and 223, the user can detach the second housing 210b from the first housing 210a. In contrast, if a user positions the second housing 210b relative to the first housing 210a, and tightens the screws 221, 222, and 223 via holes 221b, 222b (not illustrated), and 223c (not illustrated) provided in the second housing 210b, the user can attach the second housing 210b to the first housing 210a.

As described above, an operation of detaching the second housing 210b includes a step of pulling the protruding portion 290 from the ring member 280 toward the negative side in the X2 direction. An operation of attaching the second housing 210b includes a step of inserting the protruding portion 290 into the ring member 280 toward the positive side in the X2 direction.

The protruding portion 290 inserted into the ring member 280 as described prevents portions of the first housing 210a and the second housing 210b, which are positioned closer to the positive side in the X2 direction than the screw 221, from separating from each other in the Y direction. For this reason, even if the vicinity of a distal end of the housing 210 is not tightened with screws, the first housing 210a and the second housing 210b can be reliably joined together. The tip end referred to here is a positive side end portion in the X1 direction, and is a positive side end portion in the X2 direction.

Since the distal end is joined via configuration elements which are provided in the first housing 210a and the second housing 210b as described above, it is possible to avoid an increase in the number of components, and to avoid a risk of additional joining components falling off during surgery.

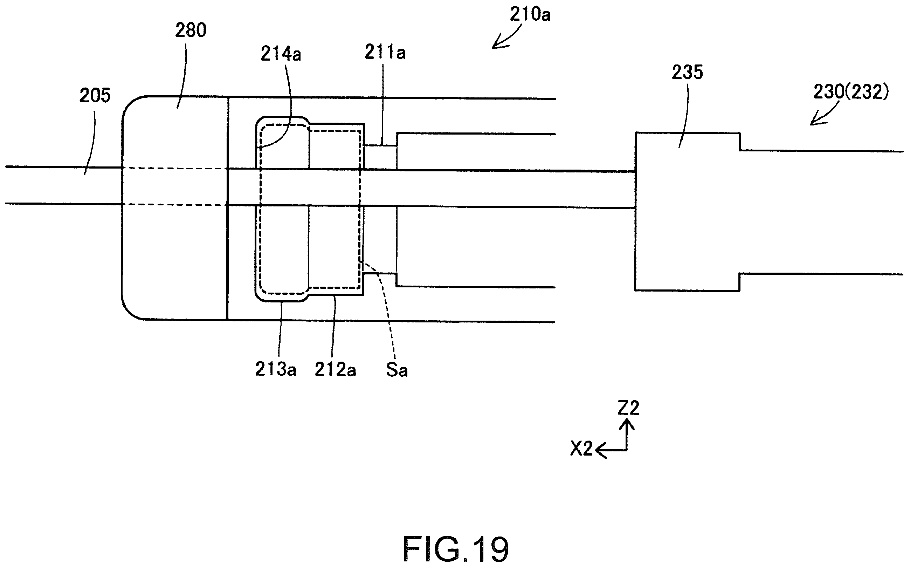

The aspirating flow path member 230 includes a head portion 235 (to be described later with reference to Fig. 19) in a positive side end portion thereof in the X2 direction. In a state where the second housing 210b is detached from the first housing 210a, a half of the head portion 235 is accommodated in an accommodation space Sa (to be described later with reference to Fig. 19). The half of the head portion 235 referred to here is a portion that is positioned on the negative side in the Y direction between portions into which the head portion 235 is bisected by a Z2-X2 plane.

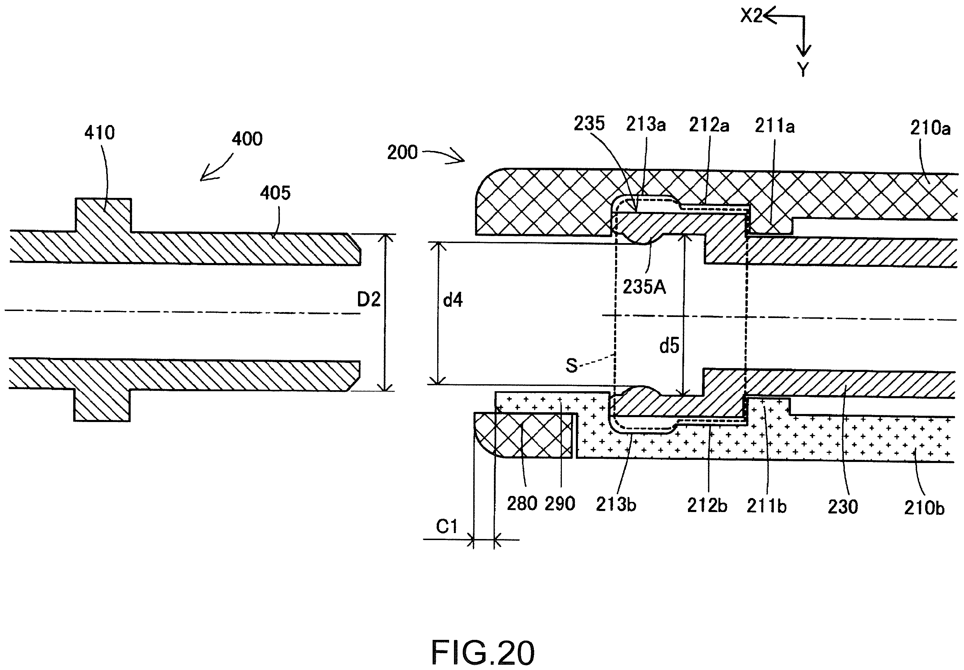

In contrast, an accommodation space Sb is provided in the second housing 210b. In a state where the second housing 210b is attached to the first housing 210a, the head portion 235 is accommodated in a space S formed of the accommodation space Sa and the accommodation space Sb (refer to Fig. 20).

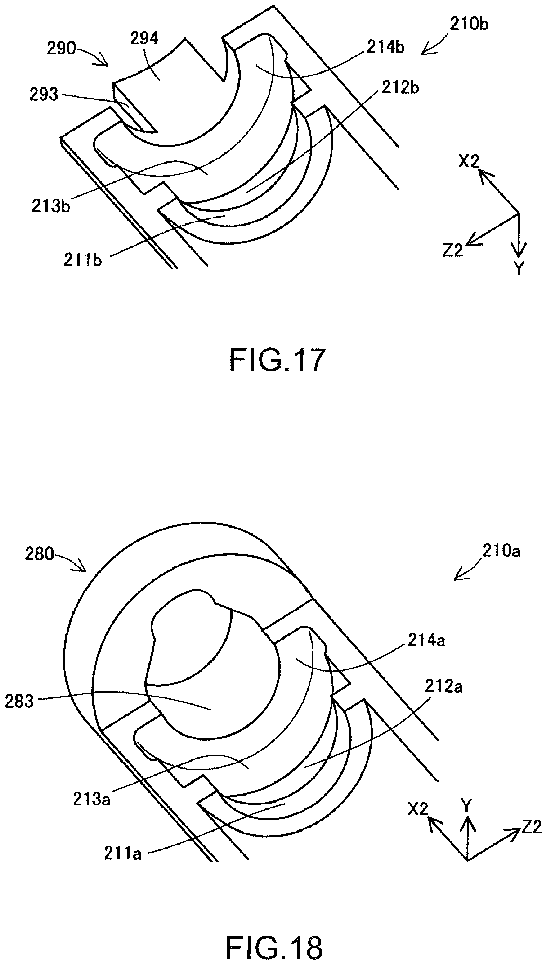

Figs. 16 and 17 are perspective view illustrating the vicinity of the protruding portion 290 of the second housing 210b. The protruding portion 290 includes the distal end surface 291; an inclined surface 292; an outer peripheral surface 293; and an inner peripheral surface 294. As illustrated in Fig. 11, the shapes of the outer peripheral surface 293 and the inner peripheral surface 294 are determined such that the outer peripheral surface 293 and the inner peripheral surface 294 are received by the indented surface 285. The outer peripheral surface 293 is formed of five flat surfaces. The inner peripheral surface 294 is formed of an arc surface. The distal end surface 291 is a portion of the second housing 210b which is positioned farthest to the positive side in the X2 direction.

The inclined surface 292 is a portion formed by chamfering the distal end surface 291 and the surfaces of the outer peripheral surface 293. Since the inclined surface 292 is formed, the protruding portion 290 is easily inserted into the indented surface 285.

As illustrated in Fig. 17, a stopper 211b, a small-diameter portion 212b, a large-diameter portion 213b, and a wall 214b are formed in the second housing 210b. The stopper 211b, the small-diameter portion 212b, the large-diameter portion 213b, and the wall 214b form a portion of a boundary surface of the accommodation space Sb described with reference to Fig. 15. The accommodation space Sb is a space that is partitioned off by the boundary and virtual blocking of through holes that open in the stopper 211b and the wall 214b.

Fig. 18 is a perspective view of the vicinity of the ring member 280 of the first housing 210a. As illustrated in Fig. 18, a stopper 211a, a small-diameter portion 212a, a large-diameter portion 213a, and a wall 214a are formed in the first housing 210a. The stopper 211a, the small-diameter portion 212a, the large-diameter portion 213a, and the wall 214a form a portion of a boundary surface of the accommodation space Sa described with reference to Fig. 15. The accommodation space Sa is a space that is partitioned off by the boundary and virtual blocking of through holes that open in the stopper 211a and the wall 214a. In this configuration, the stopper 211a, the small-diameter portion 212a, the large-diameter portion 213a, and the wall 214a are shaped symmetrically to the stopper 211b, the small-diameter portion 212b, the large-diameter portion 213b, and the wall 214b of the second housing 210b.

Fig. 19 illustrates a state in which the aspirating flow path member 230 is detached from the first housing 210a and the supply pipe 205 passes through the ring member 280. The aspirating flow path member 230 includes the head portion 235. The head portion 235 is formed on the farthest side of the aspirating flow path member 230 which is positioned on the positive side in the X2 direction, that is, on the farthest side of the distal end side 232 which is positioned on the positive side in the X2 direction. The outer diameter of the head portion 235 is larger than those of other portions of the aspirating flow path member 230. The outer diameter of the head portion 235 is larger than the inner diameter of the stopper 211a, and is smaller than the inner diameters of the small-diameter portion 212a and the large-diameter portion 213a.

When a user attaches the aspirating flow path member 230 to the first housing 210a, the user brings the aspirating flow path member 230 close to the ring member 280, and gets a half of the head portion 235 accommodated in the space Sa. Therefore, movement of the head portion 235 to the positive side in the X2 direction is restricted by the wall 214a. Movement of the head portion 235 to the negative side in the X2 direction is restricted by the stopper 211a. Movement of the head portion 235 to the negative side in the Y direction or to the Z2 direction is restricted by the small-diameter portion 212a and the large-diameter portion 213a.

When the second housing 210b having the same structure is fixed to the first housing 210a, movement of the head portion 235 to a positive side in the Y direction is restricted by the small-diameter portion 212b and the large-diameter portion 213b. As a result, the head portion 235 is held inside of the accommodation space S.

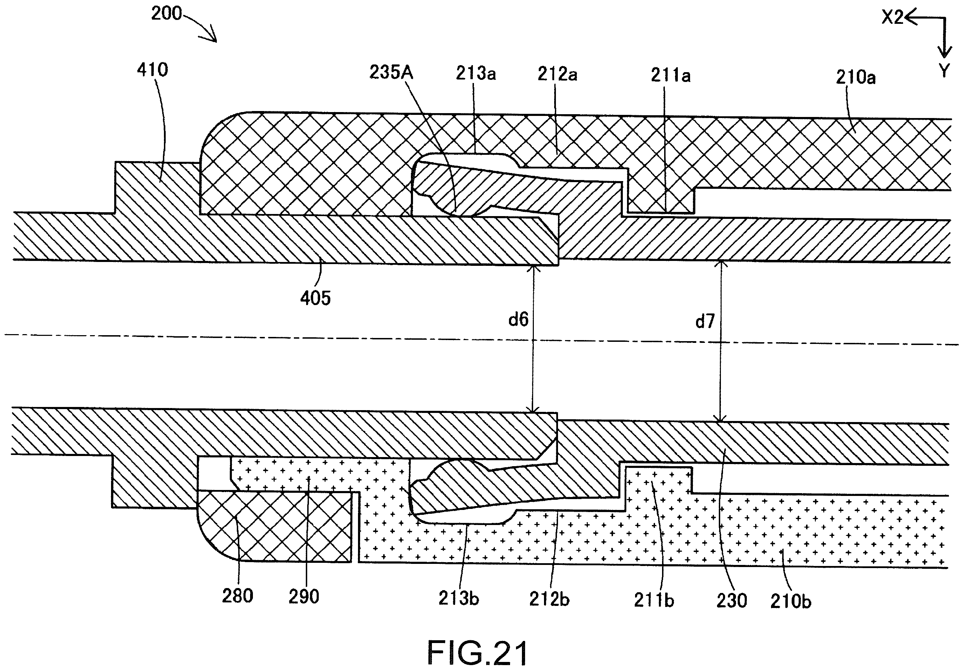

Fig. 20 is a sectional view of the handpiece 200 and the aspiration pipe 400 which are disassembled from each other. Fig. 21 is a sectional view of the handpiece 200 in a mounting state. Sealing between the aspiration pipe 400 and the aspirating flow path member 230 will be described with reference to Figs. 20 and 21.

The head portion 235 includes a sealing portion 235A. The sealing portion 235A is a portion of the head portion 235 in a mounting state which is bulged from an inner wall of the head portion 235 toward the inserted portion 405. That is, the sealing portion 235A is a portion that is bulged from the inner wall of the head portion 235 toward a central axis line of the head portion 235.

The inner diameter of the narrowest portion of the sealing portion 235A is d4. The sealing portion 235A is positioned closer to the positive side in the X2 direction than the center of the head portion 235. For this reason, the sealing portion 235A is disposed in the range of the large-diameter portions 213a and 213b in the X2 direction. The inner diameter of the widest portion of the head portion 235 is d5. The inner diameter d5 is larger than the outer diameter D2 of the inserted portion 405.

Since the aforementioned dimensional relationship is established, if the inserted portion 405 is inserted into the head portion 235, interference fitting therebetween is established. That is, if the inserted portion 405 is inserted into the head portion 235, as illustrated in Fig. 21, radially outward force is applied to the sealing portion 235A, and the head portion 235 is deformed. A gap between the aspiration pipe 400 and the aspirating flow path member 230 is sealed by reaction force induced by the deformation and contact pressure between the sealing portion 235A and an outer peripheral surface of the inserted portion 405.

The reason why the large-diameter portions 213a and 213b are provided is to prevent contact between the head portion 235 and the housing 210 from hindering the aforementioned deformation. For this reason, the range of the large-diameter portions 213a and 213b in the X2 direction includes the position of the sealing portion 235A in the X direction (the direction of insertion).

In contrast, the reason why the small-diameter portions 212a and 212b are provided is to stabilize the radial position of the head portion 235, and to prevent an excessive radial increase of the head portion 235 in a mounting state.

A user can correctly mount the aspiration pipe 400 by inserting the aspiration pipe 400 until the flange 410 comes into contact with the ring member 280. At this time, the outer peripheral surface of the inserted portion 405 comes into contact with only the sealing portion 235A. Accordingly, there is almost no change in the area of contact between the inserted portion 405 and the head portion 235 regardless of the depth of insertion, and the area of contact is constant. For this reason, there is almost no change in frictional force between the inserted portion 405 and the head portion 235 regardless of the depth of insertion. For this reason, a mounting failure is unlikely to occur. The mounting failure referred to here implies that an inserting force is increased as the depth of insertion is increased, and a user may stop inserting the inserted portion 405 before the flange 410 comes into contact with the ring member 280.

The inner diameter of the aspiration pipe 400 is d6. The inner diameter of aspirating flow path member 230 is d7. The inner diameter d7 is larger than the inner diameter d6. That is, the flow path diameter of the aspirating flow path member 230 is larger than that of the aspiration pipe 400. For this reason, when solid matter and the like, which have flowed through the aspiration pipe 400, flow into the aspirating flow path member 230, the solid matter and the like are prevented from being stuck, and an aspiration flow becomes smooth.

The invention is not limited to the embodiment, examples, and modification examples of the specification, and can be realized with various configurations insofar as the various configurations do not depart from the concept of the invention. For example, technical characteristics of the embodiment, examples, and modification examples which correspond to technical characteristics of each aspect described in SUMMARY may be suitably replaced or combined together so as to solve a portion or the entirety of the aforementioned task, or to achieve a portion or the entirety of the aforementioned effects. If the technical characteristics are not described as being necessary in the specification, the technical characteristics may be suitably eliminated. Hereinafter, examples will be described.

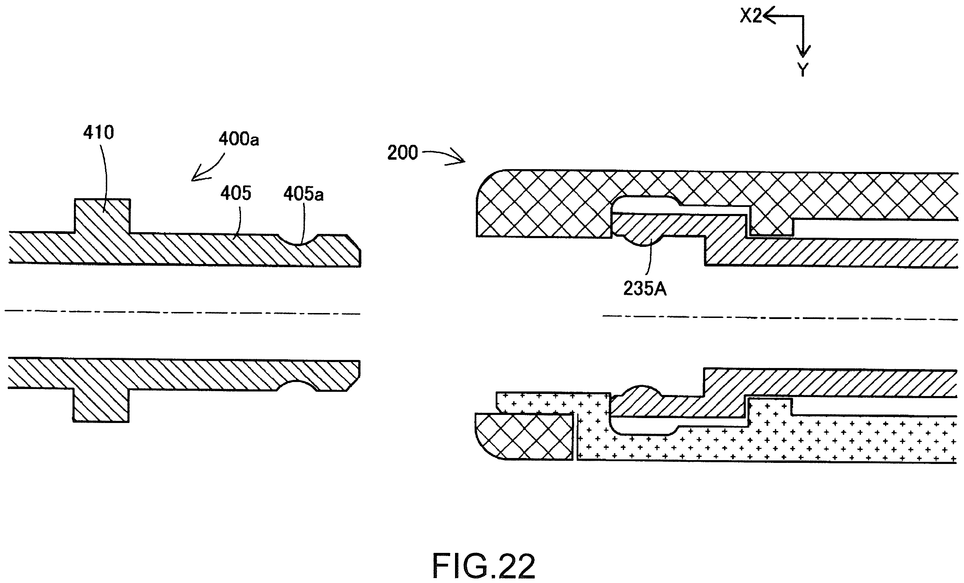

Modification Example 1 will be described. Fig. 22 illustrates sections of a aspiration pipe 400a and the handpiece 200 which are disassembled from each other. The aspiration pipe 400a is used instead of the aspiration pipe 400 of the embodiment. The handpiece 200 is the same as that of the embodiment. The inserted portion 405 of the aspiration pipe 400a includes a recess 405a. The recess 405a is a circular annular recess provided on the outer periphery of the inserted portion 405. The section of the recess 405a has an arc shape.

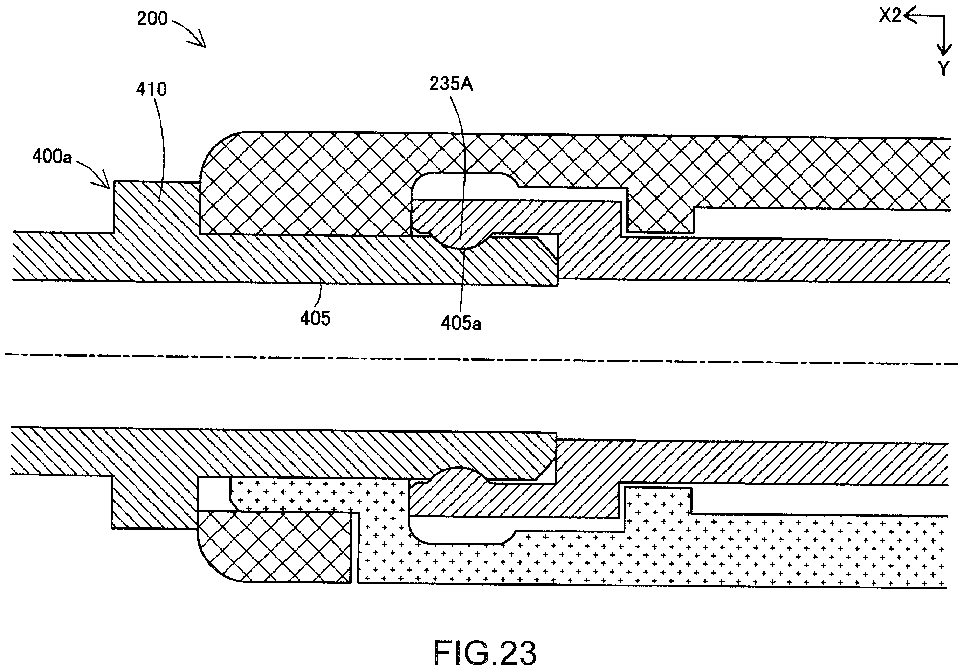

Fig. 23 illustrates the sections of the aspiration pipe 400a and the handpiece 200 which are mounted to each other. If the aspiration pipe 400a is used, and the inserted portion 405 is inserted to a correct position, the sealing portion 235A is fitted to the recess 405a. For this reason, if the inserted portion 405 is inserted to the correct position, a user feels "click". Accordingly, the user easily tactually determines that the aspiration pipe 400a is correctly mounted.

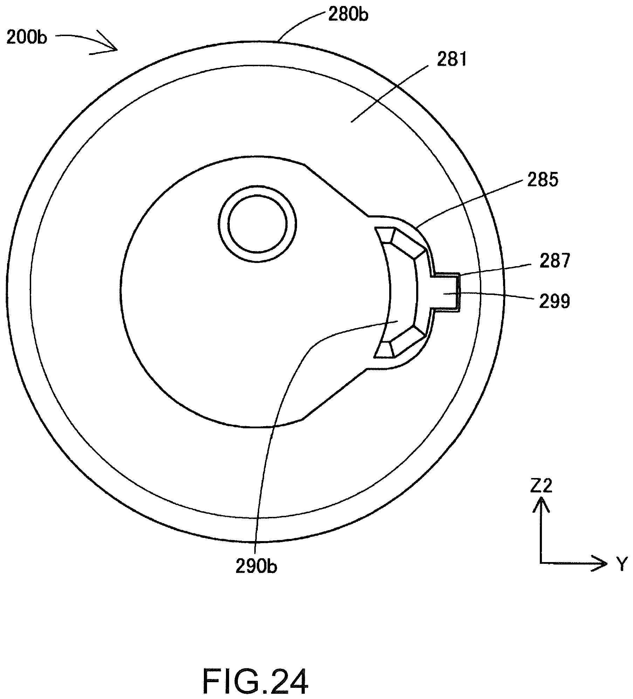

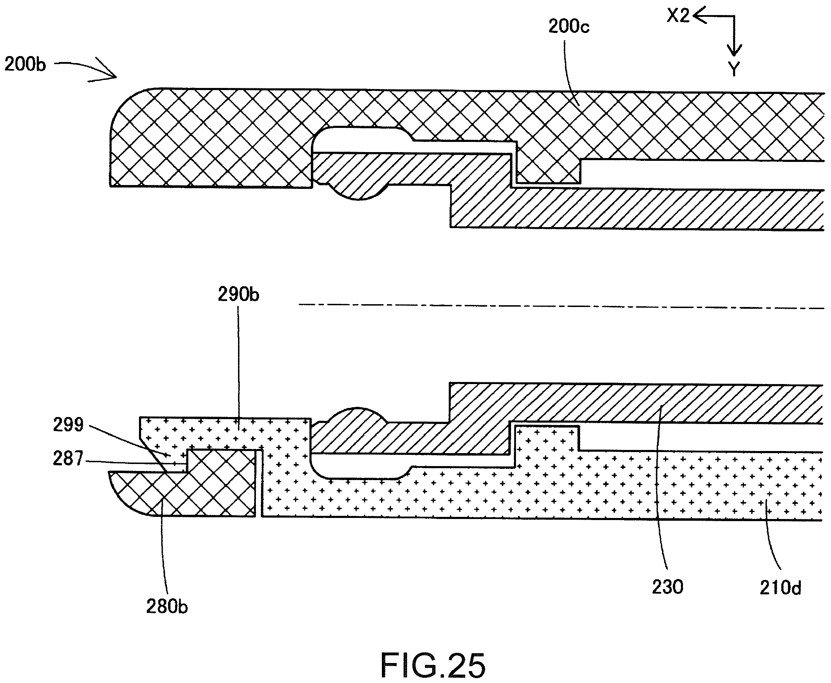

Modification Example 2 will be described. Fig. 24 is a view of the connection portion 10 viewed from the positive side toward the negative side in the X2 direction. Fig. 25 is a sectional view of the vicinity of the connection portion 10 of a handpiece 200b. The handpiece 200b is used instead of the handpiece 200 of the embodiment.

The handpiece 200b includes a first housing 200c and a second housing 200d. The first housing 200c includes a ring member 280b. The ring member 280b includes a stepped portion 287. The stepped portion 287 is a portion of the indented surface 285. As illustrated in Fig. 25, the stepped portion 287 is a portion that forms a step in the indented surface 285, and forms a surface parallel to the Y-Z2 plane.

The second housing 200d includes a protruding portion 290b. The protruding portion 290b includes a hook portion 299. The hook portion is a portion that protrudes from an outer peripheral surface of the protruding portion 290b to the positive side in the Y direction.

As illustrated in Fig. 25, in a state where the first housing 200c and the second housing 200d are joined together, the hook portion 299 is hooked onto the stepped portion 287. For this reason, even if the screws 221, 222, and 223 are loosened, there is a low possibility that the first housing 200c and the second housing 200d are separated from each other.

Hereinafter, other modification examples will be described.

A liquid ejection configuration, in which a liquid is irradiated with electromagnetic waves such as optical masers or laser beams or a liquid is heated with a heater or the like so as to intermittently eject the liquid, may be used.

The surgical apparatus 20 may be a type of apparatus for performing surgery via a liquid, which breaks up biological tissues using ultrasonic waves. Such a surgical apparatus also may adopt the same configuration described in the embodiment in which a liquid is supplied from the supply pipe 205 and the liquid or resected biological tissues are aspirated via the aspiration pipe 400.

The shapes of the ring member 280 and the inserted portion 405 may be changed while maintaining a feature that the aspiration pipe 400 can be attached to and detached from the housing 210. For example, the virtual inscribed circle V may be in contact with the through hole 289 of the ring member 280 at four or more points.

A configuration, in which the protruding portion 290 is disposed inside of the indented surface 285 in a mounting state, may be changed. For example, the indented surface 285 as a configuration element of the ring member 280 may not be provided. The protruding portion may be in contact with the inserted portion 405 in a mounting state.

The distal end surface of the inserted portion 405 may not be chamfered.

The aspiration pipe 400 may not include the flange 410. Even if the flange 410 is not provided, a user can correctly mount the aspiration pipe 400 by inserting the aspiration pipe 400 until the distal end surface of the inserted portion 405 comes into contact with the head portion 235.

The distal end surface 291 of the protruding portion 290 may not be chamfered.

The distal end surface 291 of the protruding portion 290 may protrude outward from the distal end surface 281 of the ring member 280.

The ring member 280 and the protruding portion 290 may be eliminated, and the first housing 210a and the second housing 210b may be formed into a symmetrical shape in the connection portion 10. In this case, the first housing 210a may have a shape in which the ring member 280 is cut away from the first housing 210a. The second housing 210b may has a shape in which the protruding portion 290 is cut away from the second housing 210b. In addition, end portions of the first housing 210a and the second housing 210b may be covered with a ring-shaped separate member so as to join together the end portions of the first housing 210a and the second housing 210b.

The supply pipe 205 may not pass through the ring member 280. In this case, the nozzle 207 which is an end portion of the supply pipe 205 may be disposed inside of the ring member 280.

The handpiece 200 may not include the supply pipe 205. If the supply pipe 205 is not held, the surgical apparatus 20 may be used as an aspiration apparatus.

In a two-layer pipe formed of the aspirating flow path member 230 and the supply pipe 205, the supply pipe 205 may be an outer pipe member, and the aspirating flow path member 230 may be an inner pipe member.

The angle formed by the holding portion 233 and the outer pipe member may not be the same as the angle formed by the distal end side 205b2 and the rear end side 205a2.

The inner pipe member may be formed of an insulative material.

The insulative member 270 may not be provided.

In interference fitting between the inserted portion 405 and the head portion 235, a flow path of the aspirating flow path member 230 which serves as a shaft is fitted to the aspiration pipe 400 serving as a hole. In this case, the sealing portion 235A provided in the head portion 235 may be provided on an outer peripheral surface of the head portion 235.

Instead of the sealing portion 235A, a sealing portion may be provided in the inserted portion 405. The sealing portion provided in the inserted portion 405 is a portion that is bulged toward the head portion 235, that is, a mating member of interference fitting. That is, if the inserted portion 405 serves as a shaft of interference fitting as in the embodiment, the sealing portion is provided on the outer peripheral surface of the inserted portion 405, and if the inserted portion 405 serves as a hole of the interference fitting unlike the embodiment, the sealing portion is provided on an inner peripheral surface of the inserted portion 405.

In addition to the sealing portion 235A, the aforementioned sealing portion may be provided on the outer peripheral surface of the inserted portion 405.

The head portion 235 may not be accommodated in the accommodation space S. For example, the head portion 235 may be disposed outside of the ring member 280.

The configuration in which a liquid is intermittently ejected is adopted in the embodiment. Alternatively, a configuration in which a liquid is continuously ejected may be adopted. For example, a configuration in which intermittent ejection and continuous ejection can be properly used may be adopted. In order to perform continuous ejection using a hardware configuration of the embodiment, only the tube pump may be driven in a state where the driving of the drive unit is stopped or decreased. In this configuration, intermittent ejection may be performed for resection, and continuous ejection may be performed for cleaning.

Alternatively, a configuration in which only continuous ejection can be performed may be adopted. In this configuration, resection may be performed by continuous ejection.

A liquid to be ejected may be pure water or a drug solution.

A portion or the entirety of functions and processes realized by software in the aforementioned description may be realized by hardware. A portion or the entirety of functions and processes realized by hardware may be realized by software. Various circuits such as an integrated circuit, a discrete circuit, and a circuit module which is a combination of these circuits may be used as hardware.

## Claims

1. A surgical handpiece comprising:
a housing adapted to be gripped by a user; and
an aspiration pipe that is attachably and detachably held by the housing, and aspirates a liquid.

2. The surgical handpiece according to claim 1,
wherein the housing includes a ring member in which a through hole is formed, and
wherein an inserted portion which is a portion of the aspiration pipe is inserted into the through hole such that the aspiration pipe is mounted to the housing.

3. The surgical handpiece according to claim 2,
wherein the diameter of an inscribed circle, which is virtually in contact with the through hole which is projected to a plane perpendicular to an inserting direction of the inserted portion in a state where the aspiration pipe is detached, is smaller than that of the inserted portion.

4. The surgical handpiece according to claim 3,
wherein the through hole on a projected plane includes an arc and two straight lines which are respectively connected to both ends of the arc, and
wherein the inscribed circle is in contact with the arc and the two straight lines.

5. The surgical handpiece according to claim 4,
wherein the through hole on the projected plane includes an indented surface that is connected to ends of the two straight lines and is indented toward an outer peripheral surface of the ring member.

6. The surgical handpiece according to any one of claims 2 to 5,
wherein an end surface of the inserted portion in the inserting direction is chamfered.

7. The surgical handpiece according to any one of claims 2 to 6,
wherein the aspiration pipe includes a flange which has an outer diameter larger than that of the inserted portion, and which is in contact with the housing in a state where the aspiration pipe is mounted to the housing.

8. The surgical handpiece according to any one of the preceding claims, further comprising:
a supply pipe configured to supply the liquid to a target resection site.

9. The surgical handpiece according to any one of the preceding claims,
wherein the supply pipe intermittently ejects the liquid.

10. A liquid ejecting apparatus comprising:
the surgical handpiece according to any one of the preceding claims.

11. An aspiration apparatus comprising:
the surgical handpiece according to any one of the preceding claims.
